# FASCICULE DE BREVET EUROPEEN

(11) **EP 1 450 750 B1**
(45) Date de publication et mention de la délivrance du brevet: **21.10.2009**
(21) Numéro de dépôt: 02793210.2
(22) Date de dépôt: 07.11.2002
(51) Int. Cl.: C07D 417/06, A61K 31/4178

(54) **DERIVES DE 2-AMINO-4-PYRIDYLMETHYL-THIAZOLINE ET LEUR UTILISATION COMME INHIBITEURS DE NO-SYNTHASE INDUCTIBLE**
2-AMINO-4-PYRIDYLMETHYL-THIAZOLINDERIVATE UND IHRE VERWENDUNG ALS INDUZIERBARE NO-SYNTHASE INHIBITOREN
USE OF 2-AMINO-4-PYRIDYLMETHYL-THIAZOLINE DERIVATIVES AS INHIBITORS OF INDUCIBLE NO-SYNTHASE

(30) Priorité: 09.11.2001 FR 0114508; 30.01.2002 US 352978 P
(43) Date de publication de la demande: 01.09.2004
(73) Titulaire: Aventis Pharma S.A., 92160 Antony (FR)
(72) Inventeur: BACQUE, Eric, F-91190 Gif sur Yvette (FR); BIGOT, Antony, F-91300 Massy (FR); CARRY, Jean-Christophe, F-94100 Saint Maur des fosses (FR); MIGNANI, Serge, F-92290 Chatenay-Malabry (FR); RONAN, Baptiste, F-92140 Clamart (FR); TABART, Michel, F-91290 La Norville (FR)
(74) Mandataire: Rousseau, Pierrick Edouard
(86) Numéro de dépôt international: PCT/FR2002/003808
(87) Numéro de publication internationale: WO 2003/039446

(56) Documents cités:
- WO-A-01/94325
- WO-A-94/12165
- WO-A-95/11231
- WO-A-96/14842
- HARRIS R L N ET AL: "Potential wool growth inhibitors. 2(1H)-Pyridinone analogues of mimosine" AUSTRALIAN JOURNAL OF CHEMISTRY, vol. 30, no. 3, 1977, pages 649-655, XP008005775
- ADAMCZYK M ET AL: "Nonproteinogenic amino acids: an efficient asymmetric synthesis of (S)-(-)-acromelobic acid and (S)-(-)-acromelobinic acid" TETRAHEDRON, ELSEVIER SCIENCE PUBLISHERS, AMSTERDAM, NL, vol. 58, no. 34, août 2002 (2002-08), pages 6951-6963, XP004377348 ISSN: 0040-4020

## Description

La présente invention concerne l'utilisation de dérivés de 2-amino-4-pyridylméthylthiazoline de formule (I): 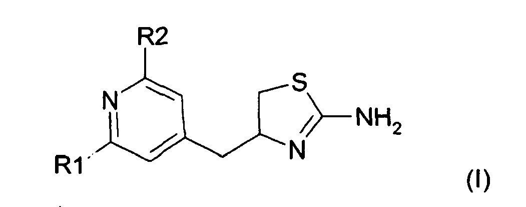 ou leurs sels pharmaceutiquement acceptables comme inhibiteurs de NO-synthase inductible.

L'invention a pour objet l'utilisation des dérivés de 2-amino-4-pyridylméthylthiazoline de formule (I) et leurs sels pharmaceutiquement acceptables pour la préparation de compositions pharmaceutiques destinées à prévenir et traiter les maladies dans lesquelles une production anormale de monoxyde d'azote (NO) par induction de NO-synthase inductible (NOS-2 ou iNOS) est impliquée, les compositions pharmaceutiques contenant les nouveaux dérivés de 2-amino-4-pyridylméthyl-thiazoline et leurs sels pharmaceutiquement acceptables et les dérivés nouveaux de 2-amino-4-pyridylméthyl-thiazoline et leurs sels pharmaceutiquement acceptables..

Le monoxyde d'azote (NO) est un radical diffusible impliqué dans de nombreux processus physiologiques et pathologiques. Il est synthétisé par oxydation de la L-Arginine, une réaction catalysée par une famille d'enzymes appelée synthase du monoxyde d'azote ou NO-Synthase (NOS), référencée dans le système international de nomenclature des enzymes sous le numéro E.C. 1.14.13.39.

Trois isoformes de NOS, dont deux sont constitutives et une inductible, sont connues :
- une NOS neuronale (NOS-1 ou nNOS) a été isolée et clonée à l'origine à partir de tissu nerveux où c'est une enzyme constitutive. La NOS-1 produit du NO en réponse à divers stimuli physiologiques tels que l'activation de récepteurs membranaires selon un mécanisme dépendant du calcium et de la calmoduline.
- une NOS inductible (NOS-2 ou iNOS) peut être induite en réponse à des stimuli immunologiques tels que par exemple des cytokines ou des antigènes bactériens dans différentes cellules tels que par exemple les macrophages, les cellules endothéliales, les hépatocytes, les cellules gliales, ainsi qu'un grand nombre d'autres types de cellules. L'activité de cette isoforme n'est pas régulée par le calcium. C'est pourquoi une fois induite elle produit de grandes quantités de NO sur des durées prolongées.
- une NOS endothéliale (NOS-3 ou eNOS) est constitutive et calcium/calmoduline dépendante. Elle a été identifiée à l'origine dans les cellules de l'endothélium vasculaire où elle génère du NO en réponse à des stimuli physiologiques tels que l'activation de récepteurs membranaires.

Le NO produit par les isoformes constitutives neuronales et endothéliales (NOS-1 et NOS-3) est généralement impliqué dans des fonctions de signalisation intercellulaire. Par exemple, les cellules endothéliales qui tapissent la paroi interne des vaisseaux sanguins induisent la relaxation des cellules musculaires lisses sous-jacentes *via* la production de NO. Il contribue ainsi à la régulation de la pression artérielle.

Le NO produit en grande quantité par l'isoforme inductible NOS-2 est, entre autre, impliqué dans les phénomènes pathologiques associés aux processus inflammatoires aigus et chroniques dans une grande variété de tissu et d'organes.

Une production excessive de NO par induction de NOS-2 participe ainsi de pathologies dégénératives du système nerveux comme par exemple la sclérose en plaques, l'ischémie cérébrale focale ou globale, les traumatismes cérébraux ou spinaux, la maladie de Parkinson, la maladie de Huntington, la maladie d'Alzheimer, la sclérose latérale amyotrophique, la migraine, la dépression, la schizophrénie, l'anxiété, l'épilepsie. De même, en dehors du système nerveux central, l'induction de NOS-2 est impliquée dans de nombreuses pathologies à composantes inflammatoires comme par exemple le diabète, l'athérosclérose, la myocardite, l'arthrite, l'arthrose, l'asthme, le syndrome du colon irritable, la maladie de Crohn, la péritonite, le reflux gastro-oesophagien, l'uvéite, le syndrome de Guillain-Barré, la glomérulo-néphrite, le lupus érythémateux, le psoriasis. La NOS-2 a également été impliquée dans la croissance de certaines formes de tumeurs comme par exemple des épithéliomes, des adénocarcinomes ou des sarcomes, et dans les infections par des bactéries intracellulaires ou extracellulaires, Gram-plus ou Gram-moins.

Dans toutes les situations où une surproduction de NO est néfaste, il apparaît donc souhaitable de diminuer la production de NO par l'administration de substances capables d'inhiber la NOS-2. Cependant, compte tenu des rôles physiologiques importants joués par l'isoforme constitutive NOS-3 en particulier dans la régulation de la pression artérielle, il est primordial que l'inhibition de l'isoforme NOS-2 affecte le moins possible l'isoforme NOS-3. En effet, il est connu que l'administration d'inhibiteurs non-sélectifs des isoformes de NOS conduit à une vasoconstriction et à un accroissement de la pression artérielle (Moncada, S., Palmer, R.M.J. et Higgs, E.A., Biosynthesis of nitric oxide from L-arginine : a pathway for the regulation of cell function and communication, Biochem. Pharmacol., 1989, 38: 1709-1715). Ces effets sur le système cardiovasculaire sont délétères dans la mesure où ils diminuent l'apport en nutriments aux tissus. Par conséquent, la présente invention concerne des composés présentant une activité inhibitrice vis-à-vis de la NOS-2 significativement plus puissante que son activité inhibitrice vis-à-vis de la NOS-3.

Des inhibiteurs de NOS dérivés de thiazoline sont notamment décrits dans les demandes de brevet WO94/1216 WO95/1123 et WO96/14842.

La présente invention concerne l'utilisation des dérivés de 2-amino-4-pyridylméthylthiazoline de formule (I) dans laquelle :
soit R1, R2 sont identiques et représentent un radical hydroxy, alkyle(C1-C4) un chlore ou un alcoxy (C1-C4) ;
soit au moins un des deux R1, R2 est un hydrogène et l'autre est un radical alkyle(C1-C4), alcoxy(C1-C4), un hydroxy ou un chlore
pour la préparation de médicaments utiles pour prévenir ou traiter les maladies dans lesquelles une production anormale de monoxyde d'azote (NO) par induction de NO-synthase inductible (NOS-2 ou iNOS) est impliquée.

Dans les définitions précédentes et celles qui suivent, les radicaux alkyle(C1-C4) et alcoxy(C1-C4) contiennent 1 à 4 atomes de carbone en chaîne droite ou ramifiée.

Les composés de formule (I) présentent un ou plusieurs carbones asymétriques et peuvent donc se présenter sous forme de racémique, d'énantiomères et de diastéréoisomères; ceux-ci font également partie de l'invention ainsi que leurs mélanges.

Par ailleurs les composés de formule (I) peuvent se présenter sous la forme tautomère (Ia) :

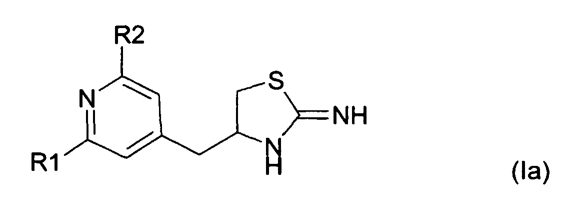

Ces tautomères font également partie de l'invention.

Parmi les composés de formule (I) utiles selon l'invention on peut citer les composés suivants:
4-(2-hydroxy-pyridin-4-ylméthyl)-4,5-dihydro-1,3-thiazol-2-ylamine
4-(2-chloro-pyridin-4-ylméthyl)-4,5-dihydro-1,3-thiazol-2-ylamine
4-(2,6-dichloro-pyridin-4-ylméthyl)-4,5-dihydro-1,3-thiazol-2-ylamine
leurs racémiques, énantiomères, diastéréoisomères, tautomères ainsi que leurs sels pharmaceutiquement acceptables,
et plus particulièrement les composés suivants :
(+)-4-(2-hydroxy-pyridin-4-ylméthyl)-4,5-dihydro-1,3-thiazol-2-ylamine
4-(2-chloro-pyridin-4-ylméthyl)-4,5-dihydro-1,3-thiazol-2-ylamine
4-(2,6-dichloro-pyridin-4-ylméthyl)-4,5-dihydro-1,3-thiazol-2-ylamine
leurs tautomères ainsi que leurs sels pharmaceutiquement acceptables.

Parmi les composés utiles selon l'invention et particulièrement préférés on peut citer le composé suivant :
4-(2-hydroxy-pyridin-4-ylméthyl)-4,5-dihydro-1,3-thiazol-2-ylamine
   son racémique, ses énantiomères, tautomères ainsi que leurs sels pharmaceutiquement acceptables,
   et plus particulièrement le composé suivant :
(+)-4-(2-hydroxy-pyridin-4-ylméthyl)-4,5-dihydro-1,3-thiazol-2-ylamine
   ses tautomères ainsi que ses sels pharmaceutiquement acceptables.

L'invention concerne également les compositions pharmaceutiques contenant en tant que principe actif un dérivé de formule (I) pour lequel soit R1, R2 sont identiques et représentent un radical hydroxy, alkyle(C1-C4) un chlore, ou un alcoxy (C1-C4) ; soit au moins un des deux R1, R2 est un hydrogène et l'autre est un radical alkyle(C1-C4), alcoxy(C1-C4), un hydroxy ou un chlore ainsi que ses racémiques, énantiomères, diastéréoisomères et leurs mélanges, leurs tautomères et ses sels pharmaceutiquement acceptables.

Les composés de formule (I) peuvent être préparés par cyclisation d'un dérivé de formule : 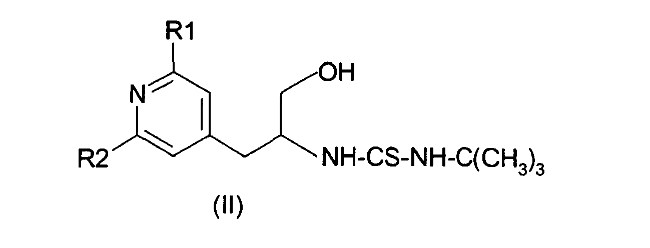 dans laquelle R₁ et R₂ ont les mêmes significations que dans la formule (I).

Cette cyclisation s'effectue généralement au moyen d'un acide tel que l'acide chlorhydrique, en milieu aqueux, à une température voisine de 100°C. De préférence, on utilise l'acide chlorhydrique 6N.

Les dérivés de formule (II) peuvent être obtenus selon le schéma réactionnel suivant : 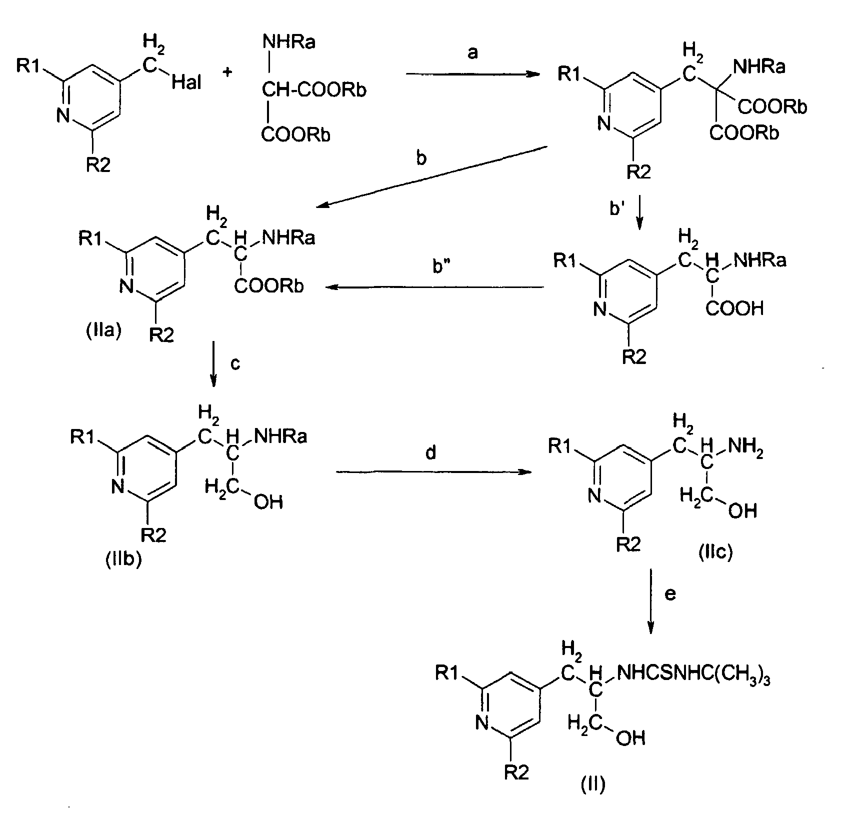 dans ces formules Ra est un groupe protecteur de la fonction amine tel que ceux décrits par T.W. GREENE, Protective groups in Organic Synthesis, J. Wiley-Interscience Publication (1991), de préférence un radical acétyle ou *tert-*butoxycarbonyle, Rb est un radical alkyle (1-4C) ou alcoxycarbonyle, de préférence, méthyle, éthyle ou isobutyloxycarbonyle et Hal est un atome d'halogène, de préférence, chlore, brome ou iode.

La réaction a s'effectue généralement en présence d'alcoolate de sodium (1-4C) (éthylate de sodium de préférence), au sein de l'alcool correspondant, à une température comprise entre 10°C et la température d'ébullition du milieu réactionnel.

La réaction b s'effectue généralement au sein d'un solvant inerte tel que le diméthylformamide en présence d'iodure de lithium, à une température comprise entre 100°C et la température d'ébullition du milieu réactionnel ou bien au sein d'un alcool aliphatique (1-4C), en présence de soude, à une température de 10 à 30°C suivie d'une neutralisation par HCl aqueux (6N à 12N de préférence) puis d'un chauffage au sein d'un solvant tel que le dioxanne ou un alcool aliphatique (1-4C) à la température d'ébullition du milieu réactionnel.

La réaction b' s'effectue de préférence au moyen d'acide chlorhydrique 12N, à une température voisine de 100°C.

La réaction b" pour les dérivés pour lesquels Rb est un radical alkyle s'effectue généralement par action d'un alcool aliphatique (1-4C) (méthanol, éthanol de préférence), en présence d'un acide minéral tel que l'acide sulfurique, à une température comprise entre 50°C et la température d'ébullition du milieu réactionnel. Pour les dérivés pour lesquels Rb est un radical isobutyloxycarbonyle, cette réaction s'effectue généralement par action de chloroformiate d'isobutyle en présence d'une base telle que la triéthylamine, au sein d'un solvant inerte tel que le tétrahydrofuranne, à une température comprise entre -20°C et 0°C.

On passe du composé IIa à IIb par l'action d'un agent de réduction. La réaction de réduction c s'effectue de préférence au moyen d'un hydrure tel que le borohydrure de sodium ou l'aluminohydrure de lithium, au sein d'un alcool aliphatique (1-4C) ou du tétrahydrofuranne, à une température comprise entre 10°C et 30°C.

On passe du composé IIb à IIc par l'action d'un agent de déprotection. La réaction de déprotection d pour les composés pour lesquels Ra est un groupe protecteur de la fonction amine s'effectue par toute méthode de déprotection connue de l'homme de l'art et notamment celles décrites par T.W. GREENE, Protective groups in Organic Synthesis, J. Wiley-Interscience Publication (1991). De préférence lorsque le groupe protecteur est un radical acétyle cette réaction s'effectue au moyen d'acide chlorhydrique aqueux, à une température voisine de 100°C. Lorsque le groupe protecteur est un radical *tert*-butoxycarbonyle cette réaction s'effectue au moyen d'acide chlorhydrique au sein du dioxanne, à une température voisine de 20°C.

La réaction e s'effectue par action du *tert*-butyl isothiocyanate, au sein d'un solvant inerte tel qu'un alcool aliphatique (1-4C) (méthanol, éthanol de préférence), éventuellement en présence d'une amine tertiaire telle que la triéthylamine, à une température comprise entre 20°C et la température d'ébullition du milieu réactionnel.

Le composé de formule (II) pour lequel R₁ et R₂ sont OH peut être préparé de la même façon à partir de composés de formule (III) pour lesquels Z est un radical silyle, de préférence *tert*-butyldiméthylsilyle, dont la préparation est décrite dans Synthesis 1994, 486 et Tetrahedron 1986, 42, 2725 .

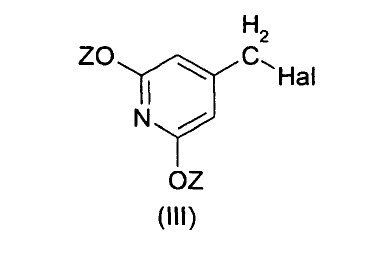

Le composé de formule (I) pour lequel R₁ est OH et R₂ est hydrogène peut être préparé selon le schéma réactionnel suivant : 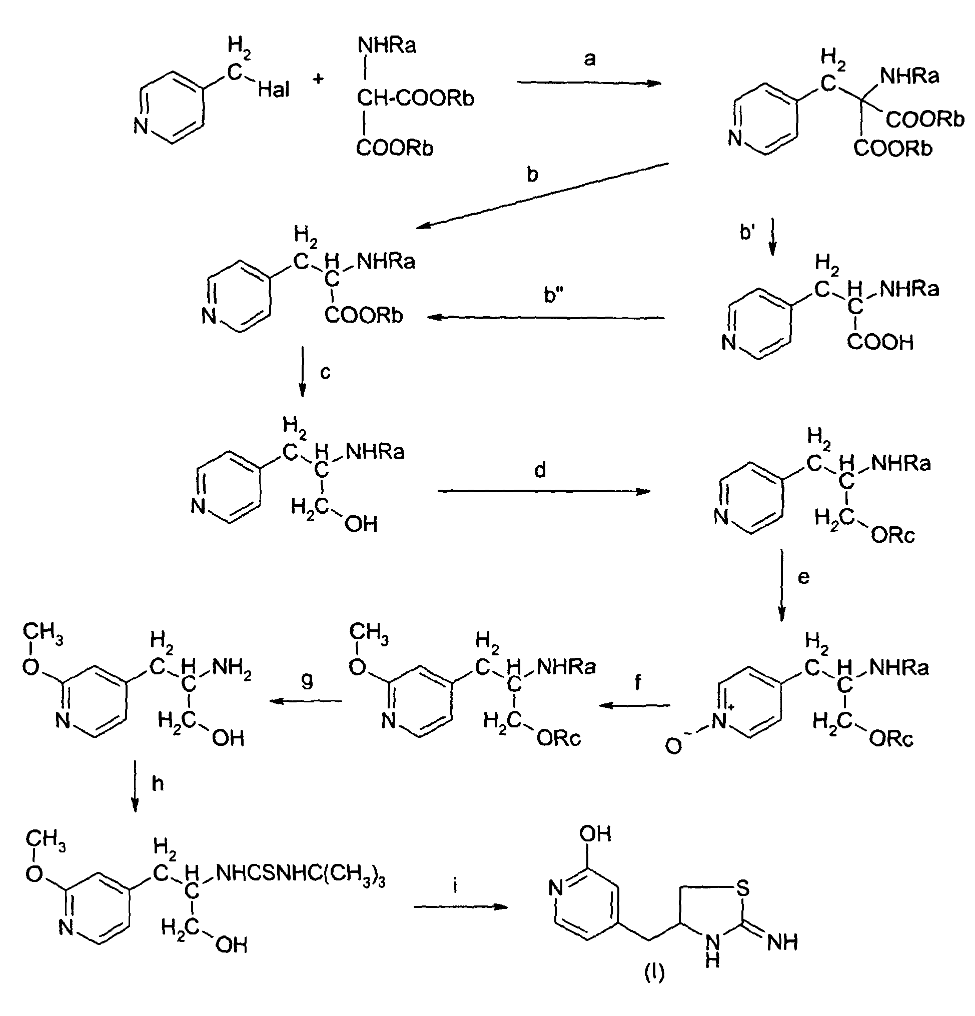 dans ces formules Ra est un groupe protecteur de la fonction amine tel que ceux décrits par T.W. GREENE, Protective groups in Organic Synthesis, J. Wiley-Interscience Publication (1991), de préférence un radical acétyle ou *tert-*butoxycarbonyle et Rb est un radical alkyle (1-4C) ou alcoxycarbonyle, de préférence, méthyle, éthyle ou isobutyloxycarbonyle. Rc est un groupe protecteur de la fonction alcool tel que ceux décrits par T.W. GREENE, Protective groups in Organic Synthesis, J. Wiley-Interscience Publication (1991), de préférence un radical silyle et plus particulièrement *tert*-butyldiméthylsilyle.

La réaction a s'effectue généralement en présence d'alcoolate de sodium (1-4C) (éthylate de sodium de préférence), au sein de l'alcool correspondant, à une température comprise entre 10°C et la température d'ébullition du milieu réactionnel.

La réaction b s'effectue généralement au sein d'un solvant inerte tel que le diméthylformamide en présence d'iodure de lithium, à une température comprise entre 100°C et la température d'ébullition du milieu réactionnel ou bien au sein d'un alcool aliphatique (1-4C), en présence de soude, à une température de 10 à 30°C suivie d'une neutralisation par HCl 6N puis d'un chauffage au sein d'un solvant tel que le dioxanne ou un alcool tel que l'éthanol à la température d'ébullition du milieu réactionnel.

La réaction b' s'effectue de préférence au moyen d'acide chlorhydrique 12N, à une température voisine de 100°C.

La réaction b" pour les dérivés pour lesquels Rb est un radical alkyle s'effectue généralement par action d'un alcool aliphatique (1-4C) (méthanol, éthanol de préférence), en présence d'un acide minéral tel que l'acide sulfurique, à une température comprise entre 50°C et la température d'ébullition du milieu réactionnel. Pour les dérivés pour lesquels Rb est un radical isobutyloxycarbonyle, cette réaction s'effectue généralement par action de chloroformiate d'isobutyle en présence d'une base telle que la triéthylamine, au sein d'un solvant inerte tel que le tétrahydrofuranne, à une température comprise entre -20°C et 0°C.

La réaction de réduction c s'effectue de préférence au moyen d'un hydrure tel que le borohydrure de sodium ou le borohydrure de lithium, au sein d'un alcool aliphatique (1-4C) et/ou du tétrahydrofuranne, à une température comprise entre 10°C et 30°C.

La réaction de protection d s'effectue par toute méthode de protection de la fonction alcool tel que celles décrites par T.W. GREENE, Protective groups in Organic Synthesis, J. Wiley-Interscience Publication (1991), de préférence au moyen de chlorure de *tert*-butyldiméthylsilyle, en présence d'une base telle qu'une amine tertiaire (diisopropyléthylamine de préférence), au sein d'un solvant tel que le dichlorométhane, à une température comprise entre 0°C et 30°C.

La réaction e s'effectue en présence d'acide 3-chloro-peroxybenzoïque, au sein d'un solvant tel que le dichlorométhane à une température comprise entre 0°C et la température d'ébullition du milieu réactionnel.

La réaction f s'effectue en présence de chlorure de *para*-tolènesulfonyle, en présence d'une base telle qu'une amine tertiaire (triéthylamine de préférence), au sein d'un solvant tel q'un alcool (méthanol de préférence) à une température comprise entre 10°C et la température d'ébullition du milieu réactionnel.

La réaction de déprotection g pour les composés pour lesquels Ra est un groupe protecteur de la fonction amine et Rc est un groupe protecteur de la fonction alcool s'effectue par toute méthode de déprotection connue de l'homme de l'art et notamment celles décrites par T.W. GREENE, Protective groups in Organic Synthesis, J. Wiley-Interscience Publication (1991). De préférence lorsque le groupe protecteur Ra est un radical acétyle et lorsque le groupe protecteur Rc est un radical silyle et plus particulièrement *tert*-butyldiméthylsilyle cette réaction s'effectue au moyen d'acide chlorhydrique aqueux (HCl 6N de préférence), à la température d'ébullition du milieu réactionnel.

La réaction h s'effectue par action du *tert*-butyl isothiocyanate, au sein d'un solvant inerte tel qu'un alcool aliphatique (1-4C) (méthanol, éthanol de préférence), éventuellement en présence d'une amine tertiaire telle que la triéthylamine, à une température comprise entre 20°C et la température d'ébullition du milieu réactionnel.

La réaction i s'effectue généralement au moyen d'un acide tel que l'acide chlorhydrique, en milieu aqueux, à la température d'ébullition voisine de 100°C du milieu réactionnel. De préférence, on utilise l'acide chlorhydrique 6N.

Les composés de formule (I) pour lesquels soit R₁ est OAlk et R₂ est hydrogène, soit R₁ et R₂ sont OAlk peuvent être respectivement préparés à partir des composés de formule (I) pour lesquels soit R₁ est OH et R₂ est hydrogène, soit R₁ et R₂ sont OH, par alkylation avec un composé de structure Hal-Alk. Hal représente un atome d'halogène (de préférence chlore, brome ou iode) et Alk a la même signification que dans la formule (I). Cette réaction s'effectue généralement au sein d'un solvant inerte tel que le diméthylformamide, le diméthylsulfoxyde, le dioxanne, le tétrahydrofuranne en présence d'un accepteur d'acide tel qu'une trialkylamine (triéthylamine par exemple), d'un hydroxyde de métal alcalin (soude, potasse par exemple) ou un hydrure de métal alcalin (hydrure de sodium par exemple), à une température comprise entre 20°C et la température d'ébullition du milieu réactionnel.

Les composés de formule (I) sont isolés et peuvent être purifiés par les méthodes connues habituelles, par exemple par cristallisation, chromatographie ou extraction.

Les énantiomères des composés de formule (I) peuvent être obtenus par dédoublement des racémiques par exemple par chromatographie sur colonne chirale selon PIRCKLE W.H. et coll., asymmetric synthesis, vol. 1, Academic Press (1983) ou par formation de sels ou par synthèse à partir des précurseurs chiraux. Les diastéréoisomères peuvent être préparés selon les méthodes classiques connues (cristallisation, chromatographie ou à partir des précurseurs chiraux).

Les composés de formule (I) peuvent être éventuellement transformés en sels d'addition avec un acide minéral ou organique par action d'un tel acide au sein d'un solvant organique tel qu'un alcool, une cétone, un éther ou un solvant chloré. Ces sels font également partie de l'invention.

Comme exemples de sels pharmaceutiquement acceptables, peuvent être cités les sels suivants : benzènesulfonate, bromhydrate, chlorhydrate, citrate, éthanesulfonate, fumarate, gluconate, iodate, iséthionate, maléate, méthanesulfonate, méthylène-bis-b-oxynaphtoate, nitrate, oxalate, pamoate, phosphate, salicylate, succinate, sulfate, tartrate, théophyllinacétate et p-toluènesulfonate.

Les composés de formule (I) sont des inhibiteurs de NO-synthase inductible ou NO-synthase de type 2 (NOS-2) et sont ainsi utiles pour la prévention et le traitement des désordres liés à une production excessive de NO telles que la sclérose en plaques, l'ischémie cérébrale focale ou globale, les traumatismes cérébraux ou spinaux, la maladie de Parkinson, la maladie de Huntington, la maladie d'Alzheimer, la sclérose latérale amyotrophique, la migraine, la dépression, la schizophrénie, l'anxiété, l'épilepsie, le diabète, l'athérosclérose, la myocardite, l'arthrite, l'arthrose, l'asthme, le syndrome du colon irritable, la maladie de Crohn, la péritonite, le reflux gastro-oesophagien, l'uveite, le syndrome de Guillain-Barré, la glomerulo-néphrite, le lupus erythémateux, le psoriasis, la croissance de certaines formes de tumeurs comme par exemple les épithéliomes, les adénocarcinomes ou les sarcomes, et les infections par des bactéries intracellulaires ou extracellulaires, gram-plus ou gram-moins.

Leurs activités en tant qu'inhibiteurs de NOS-2 et NOS-3 ont été déterminées par la mesure de la conversion de [³H]-L-arginine en [³H]-L-citrulline par, respectivement, une fraction enzymatique NOS-2 extraite de poumons de rats ou de souris préalablement traités par des lipopolysaccharides (10 mg/kg i.p. 6 heures avant le recueil de tissu) et par une préparation commerciale de NOS-3 recombinante de boeuf. Les composés ont été incubés pendant 20 à 30 minutes à 37°C en présence de 5 µM (pour activité NOS-2) ou 10 µM (pour activité NOS-3) de [³H]-L-arginine, 1 mM de NADPH, 15 µM de tétrabiopterine, 1µM de FAD, 0.1 mM de DTT dans un tampon HEPES (50 mM, pH 6,7) contenant 10 µg/ml de calmoduline et 1.25 mM de CaCl₂ lorsque l'activité NOS-3 a été mesurée. L'incubation a été arrêtée par addition de tampon HEPES froid (100 mM, pH 5,5) contenant 10 mM d'EGTA et 500 mg d'une résine cationique échangeuse d'ion (AG50W-X8, contre-ion : Na⁺) pour séparer la [³H]-L-arginine de la [³H]-L-citrulline. Après 5 min de décantation, la radioactivité restant dans la phase liquide a été mesurée dans un compteur à scintillation en présence d'un liquide scintillant approprié. Le rendement de la récupération de la L-[³H]citrulline formée a pu être estimée en utilisant de la L-[ureido-¹⁴C]-citrulline comme standard externe.

L'activité NOS-2 ou NOS-3 a été exprimée en picomole(s) de [³H]-L-citrulline formée par minute et par milligramme de protéine contenu dans le milieu réactionel.

Dans ce test sur l'enzyme NOS-2, la CI₅₀ des composés de formule (I) est inférieure ou égale à 10 µM.

La sélectivité est mesurée par le rapport CI₅₀ NOS-3 / CI₅₀ NOS-2. Cette sélectivité est supérieure à 45.

Les composés de formule (I) présentent une toxicité faible. Leur DL₅₀ est supérieure à 40 mg/kg par voie sous cutanée chez la souris.

L'exemple suivant illustre l'invention de manière non limitative.

### Exemple 1 :

### (+)-4-(2-Hydroxy-pyridin-4-ylméthyl)-4,5-dihydro-thiazol-2-ylamine

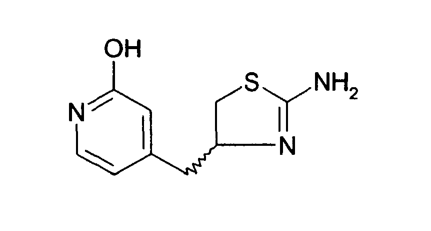

Une suspension de 0,4 g de *N*-(*tert*-butyl)-*N'*-[2-hydroxy-1-(3-méthoxypyridin-4-yl-méthyl)éthyl]-thiourée dans 4 cm³ d'une solution aqueuse d'acide chlorhydrique 6N est chauffée à une température voisine de 100°C pendant 18 heures. Le milieu réactionnel est ensuite concentré à sec sous pression réduite (2 kPa) à une température voisine de 40°C. Le résidu obtenu est repris par 25 cm³ d'eau distillée et le mélange est lavé avec 2 fois 20 cm³ de dichlorométhane. La phase aqueuse est évaporée sous pression réduite (2 kPa) à une température voisine de 40°C. Le résidu est repris avec 2 fois 15 cm³ d'éthanol et concentré à sec selon les conditions décrites ci-dessus, puis séché à l'étuve sous pression réduite (10 Pa) à une température voisine de 40°C pendant 4 heures. On obtient 0,31 g de 4-(2-amino-4,5-dihydro-thiazol-4-ylméthyl)-1H-pyridin-2-one chlorhydrate racémique, sous forme d'un solide jaune-pâle et fondant aux environs de 124°C. Ce produit est purifié par chromatographie sur une colonne CHIRALCEL OD 20µ dans un mélange heptane-éthanol-triéthylamine (80/20/0,1 en volumes). Les fractions contenant le produit attendu sont réunies et concentrées sous pression réduite (1 kPa) à une température voisine de 40°C puis le résidu est séché à l'étuve sous pression réduite (10 Pa) à une température voisine de 40°C. On obtient ainsi 0,0131 g de (+)-4-(2-Hydroxy-pyridin-4-ylméthyl)-4,5-dihydro-thiazol-2-ylamine, (α_{D}²⁰= +12,1°+/-0,7 dans le DMSO à 0,5%). Spectre de R.M.N ¹H (300 MHz, (CD₃)₂SO d6, δ en ppm) : 2,73 (dd, J = 13,5 et 7 Hz : 1H) ; 2,80 (dd, J = 13,5 et 7 Hz : 1H) ; 3.27 (dd, J = 11,5 et 5,5 Hz : 1H) ; 3,62 (dd, J = 11,5 et 7,5 Hz : 1H) ; 4,53 (mt : 1H) ; 6,13 (dd, J = 6,5 et 1,5 Hz : 1H) ; 6,24 (s large : 1H) ; 7,34 (d, J = 6,5 Hz : 1H) ; de 8,90 à 9,40 (mf très étalé : 2H) ; de 9,60 à 10,40 (mf très étalé : 1H).

### N-(tert-Butyl)-N'-[2-hydroxy-1-(3-méthoxy-pyridin-4-yl-methyl)éthyl]-thiourée

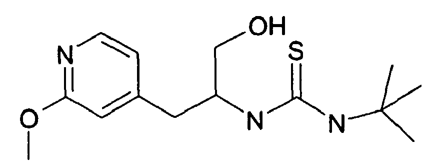

A une solution de 0,3 g de 2-amino-3-(3-méthoxy-pyridin-4-yl)-1-propanol dans 50 cm³ d'éthanol absolu, on ajoute 0,337 cm³ de *tert*-butylisothiocyanate puis le milieu réactionnel est agité sous atmosphère inerte à une température voisine de 20°C pendant 18 heures. On ajoute alors à nouveau 0,337 cm³ de *tert*-butylisothiocyanate et le mélange réactionnel est chauffé à une température voisine de 60°C pendant 4 heures. Après refroidissement, le milieu réactionnel est concentré à sec sous pression réduite (2 kPa) à une température voisine de 40°C. L'huile obtenue est purifiée par chromatographie sous pression d'argon (50 kPa), sur une colonne de gel de silice (granulométrie 40-63 µm ; diamètre 4 cm ; hauteur 22 cm), en éluant par un mélange de dichlorométhane-méthanol-ammoniaque à 20% (90/10/0,5 en volumes) et en recueillant des fractions de 10 cm³. Les fractions contenant le produit attendu sont réunies et concentrées sous pression réduite (2 kPa) à une température voisine de 40°C. On obtient ainsi 0,4 g de *N*-(*tert*-butyl)-N'-[2-hydroxy-1-(3-méthoxy-pyridin-4-yl-méthyl)éthyl]-thiourée, sous forme d'une huile jaune. Spectre infrarouge CH₂Cl₂ 3620; 3411; 2972; 1614; 1561; 1532; 1400; 1319; 1161; 1041 et 815 cm⁻¹.

### 2-Amino-3-(3-méthoxy-pyridin-4-yl)-1-propanol

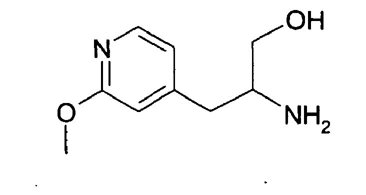

Un mélange de 1,2 g de *N*-[1-(*tert*-butyl-diméthyl-silanyloxyméthyl)-2-(3-méthoxypyridin-4-yl)éthyl]-acétamide dans 60 cm³ d'une solution aqueuse d'acide chlorhydrique 6N est chauffé à une température voisine de 100°C pendant 30 minutes. Après refroidissement à une température voisine de 20°C, on coule au goutte à goutte un volume suffisant d'une solution aqueuse de soude à 30% pour obtenir un pH voisin de 10. Le mélange est extrait par 3 fois 100 cm³ d'acétate d'éthyle et les extraits organiques sont réunis, séchés sur du sulfate de magnésium puis concentrés à sec sous pression réduite (2 kPa) à une température voisine de 40°C. On obtient ainsi 0,3 g de 2-amino-3-(3-méthoxy-pyridin-4-yl)-1-propanol, sous forme d'une huile de couleur jaune. Spectre infra-rouge CH₂Cl₂ 3628; 3335; 2948; 1614; 1561; 1400; 1319; 1162; 1044 et 815 cm⁻¹.

### N-[1-(tert-Butyl-diméthl-silanyloxyméthyl)-2-(3-méthoxy-pyridin-4-yl)éthyl]-acétamide

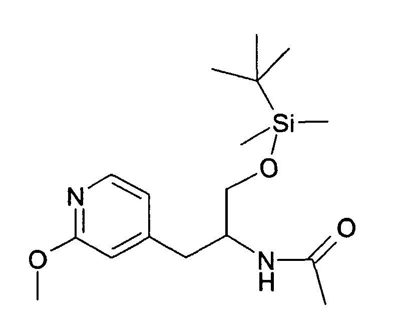

A un mélange de 5,12 g de *N*-[1-(*tert*-butyl-diméthyl-silanyloxyméthyl)-2-(1-oxy-pyridin-4-yl)éthyl]-acétamide dans 150 cm³ de méthanol anhydre, on ajoute 6 g de chlorure de *p*-toluène sulfonyle puis 8,9 cm³ de triéthylamine. Le milieu réactionnel est chauffé à une température voisine de 60°C pendant 96 heures puis concentré sous pression réduite (2 kPa) à une température voisine de 40°C. Le résidu est repris par 200 cm³ de dichlorométhane et lavé par 3 fois 150 cm³ d'eau distillée et par 100cm³ d'une solution aqueuse de chlorure de sodium saturée. La phase organique est séchée sur du sulfate de magnésium, filtrée puis concentrée sous pression réduite (2 kPa) à une température voisine de 40°C. Le résidu est purifié par chromatographie sous pression d'argon (50 kPa), sur une colonne de gel de silice (granulométrie 40-63 µm ; diamètre 7 cm ; hauteur 30 cm), en éluant par un mélange de dichlorométhane-méthanol-ammoniaque à 20% (95/5/0,5 en volumes) et en recueillant des fractions de 10 cm³. Les fractions contenant le produit attendu sont réunies et concentrées sous pression réduite (2 kPa) à une température voisine de 40°C. On obtient ainsi 1,24 g de *N*-[1-(*tert*-butyl-diméthyl-silanyloxyméthyl)-2-(3-méthoxy-pyridin-4-yl)-éthyl]-acétamide, sous forme d'une huile jaune. Spectre infra-rouge CCl₄ 3446; 2954; 2930; 2858; 1684; 1614; 1562; 1499; 1399; 1255; 1117; 1043 et 837 cm⁻¹.

### N-[1-(tert-Butyl-diméthyl-silanyloxyméthyl)-2-(1-oxy-pyridin-4-yl)-éthyl]-acétamide

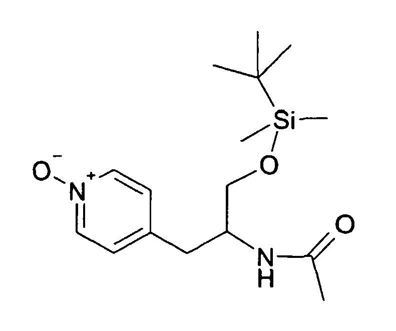

A un mélange de 21 g de *N*-[1-(*tert*-butyl-diméthyl-silanyloxyméthyl)-2-(pyridin-4-yl)-éthyl]-acétamide dans 250 cm³ de dichlorométhane sous agitation et atmosphère inerte, on ajoute progressivement 9,4 g d'acide 3-chloro-peroxy-benzoïque puis on chauffe à une température voisine de 40°C pendant 30 minutes. Le milieu réactionnel est ensuite refroidi puis lavé par 3 fois 200 cm³ d'une solution d'hydrogénocarbonate de sodium saturée puis 150 cm³ d'eau distillée. La phase organique est séchée sur du sulfate de magnésium puis concentrée à sec sous pression réduite (2 kPa) à une température voisine de 40°C. L'huile obtenue est purifiée par chromatographie sous pression d'argon (50 kPa), sur une colonne de gel de silice (granulométrie 40-63 µm ; diamètre 7 cm ; hauteur 37 cm), en éluant par des mélanges successifs de dichlorométhane-méthanol-ammoniaque à 20% (97/3/0,5, 95/5/0,5, 90/10/0,5 en volumes) et en recueillant des fractions de 50 cm³. Les fractions contenant le produit attendu sont réunies et concentrées sous pression réduite (2 kPa) à une température voisine de 40°C. On obtient ainsi 4,5 g de *N*-[1-(*tert*-butyl-diméthylsilanyloxyméthyl)-2-(1-oxy-pyridin-4-yl)-éthyl]-acétamide, sous forme d'une huile jaune. Spectre de R.M.N. ¹H (300 MHz, (CD₃)₂SO d6, δ en ppm) : 0,07 (s : 6H) ; 0,90 (s : 9H) ; 1,65 (s : 3H) ; 2,57 (dd, J = 13,5 et 9,5 Hz : 1H) ; 2,85 (dd, J = 13,5 et 4,5 Hz : 1H) ; de 3,40 à 3,60 (mt : 2H) ; 3,96 (mt : 1H) ; 7,22 (d, J = 7 Hz : 2H) ; 7,73 (d, J = 8 Hz : 1H) ; 8,12 (d, J = 7 Hz : 2H).

### N-[1-(tert-Butyl-diméthyl-silanyloxyméthyl)-2-(pyridin-4-yl)-éthyl]-acétamide

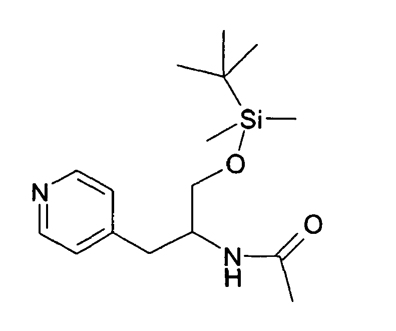

A une suspension de 25 g de *N*-(1-hydroxyméthyl-2-(pyridin-4-yl)-éthyl)-acétamide dans 700 cm³ de dichlorométhane sous agitation, on ajoute 87 g de chlorure de *ter-*butyldiméthylsilyle puis on coule goutte à goutte 112 cm³ de diisopropyléthylamine. Le milieu réactionnel est agité pendant 24 heures à une température voisine de 20°C, puis on coule de l'eau distillée. La phase organique est décantée puis lavée par 3 fois 250 cm³ d'eau distillée et 200 cm³ d'une solution aqueuse saturée en chlorure de sodium. Elle est ensuite séchée sur du sulfate de magnésium, filtrée et concentrée à sec sous pression réduite (2 kPa) à une température voisine de 40°C. L'huile obtenue est purifiée par chromatographie sous pression d'argon (50 kPa), sur une colonne de gel de silice (granulométrie 40-63 µm ; diamètre 11 cm ; hauteur 40 cm), en éluant par un mélange de dichlorométhane-méthanol-ammoniaque à 20% (95/15/0,5 en volumes) et en recueillant des fractions de 100 cm³. Les fractions contenant le produit attendu sont réunies et concentrées sous pression réduite (2 kPa) à une température voisine de 40°C. On obtient ainsi 21 g de *N*-[1-(*tert*-butyl-diméthylsilanyloxyméthyl)-2-(pyridin-4-yl)-éthyl]-acétamide, sous forme d'un solide blanc. Spectre de masse DCI m/z=309 MH⁺

### N-(1-Hydroxyméthyl-2-(pyridin-4-yl)-éthyl)-acétamide

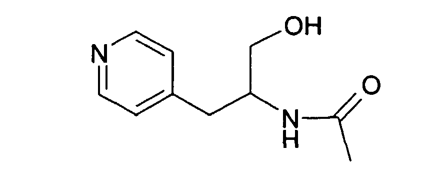

A une solution de 21,5 g de 2-(acétylamino)-3-(4-pyridinyl)propanoate d'éthyle dans 800 cm³ de méthanol anhydre, sous agitation et atmosphère inerte, on ajoute 8,7 g de borohydrure de sodium progressivement en maintenant la température inférieure à 30°C. Le milieu réactionnel est agité à une température voisine de 20°C pendant 24 heures puis est ensuite concentré sous pression réduite (2 kPa) à une température voisine de 30°C. Le résidu est repris par 450 cm³ d'eau et extrait par 4 fois 150 cm³ de dichlorométhane. La phase aqueuse est concentrée sous pression réduite (2 kPa) à une température voisine de 50°C. Le résidu est purifié par chromatographie sous pression d'argon (50 kPa), sur une colonne de gel de silice (granulométrie 40-63 µm ; diamètre 7,5 cm ; hauteur 40 cm), en éluant par un mélange de dichlorométhane-méthanol-ammoniaque à 20% (90/10/0,5 en volumes) et en recueillant des fractions de 50 cm³. Les fractions contenant le produit attendu sont réunies et concentrées sous pression réduite (2 kPa) à une température voisine de 40°C. On obtient ainsi 2,6 g de *N*-(1-hydroxyméthyl-2-(pyridin-4-yl)-éthyl)-acétamide, sous forme d'une huile qui se solidifie. Spectre IR (KBr) 3279; 3200; 2865; 1647; 1609; 1560; 1374; 1080; 1057; 1005; 907; 806; 566 et 517 cm⁻¹.

### 2-(Acétylamino)-3-(4-pyridinyl)propanoate d'éthyle

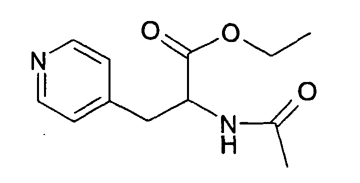

A une solution de 49,4 g de 2-(acétylamino)-2-(4-pyridinylméthyl)malonate de diéthyle dans 600 cm³ d'éthanol absolu sont additionnés goutte à goutte 51,2 cm³ d'une solution aqueuse de soude 5N. Le mélange est agité pendant 1 heure à une température voisine de 20°C puis on coule goutte à goutte 21,3 cm³ d'une solution aqueuse d'acide chlorhydrique 12N et le milieu réactionnel est chauffé à une température voisine de 70°C pendant 5 heures. Après refroidissement à une température voisine de 20°C, le mélange réactionnel est concentré sous pression réduite (2 kPa) à une température voisine de 50°C. Le résidu est repris par 200cm³ d'éthanol froid et le précipité obtenu est lavé avec 2 fois 30 cm³ d'éthanol froid. Le filtrat est évaporé selon les conditions décrites ci-dessus. Cette opération est réalisée plusieurs fois afin d'éliminer le chlorure de sodium du milieu réactionnel. On obtient ainsi 43,1 g de 2-(acétylamino)-3-(4-pyridinyl)propanoate d'éthyle, sous forme d'une huile jaune orangée. Spectre de masse EI m/z=236 M⁺ m/z=178 M - NHAc m/z=121 C₇H₉N₂⁺ m/z=93 C₆H₇N⁺ pic de base m/z=43 C₂H₃O⁺.

### 2-(Acétylamino)-2-(4-pyridinylméthyl)malonate de diéthyle

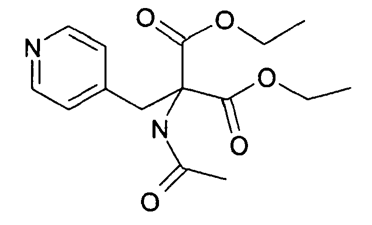

A 750 cm³ d'éthanol, on additionne par petites portions, en agitant sous atmosphère inerte, 9 g de sodium. Après consommation totale du sodium et retour à une température voisine de 20°C, on ajoute lentement 42 g d'acétamidomalonate de diéthyle puis on laisse agiter le mélange à une température voisine de 20°C pendant 1 heure 30 minutes. Une suspension de 61,5 g de bromhydrate de la 4-bromométhylpyridine dans 250 cm³ d'éthanol est ensuite coulée rapidement et la suspension résultante est agitée à une température voisine de 20°C pendant 18 heures. Le milieu réactionnel est ensuite concentré sous pression réduite (2 kPa) à une température voisine de 40°C. Le résidu est repris par 200 cm³ d'eau distillée et est neutralisé par une solution aqueuse d'acide chlorhydrique 1N jusqu'à ce que le pH soit égal à 7. La suspension obtenue est refroidie à une température voisine de 0°C, filtrée et l'insoluble est lavé avec 2 fois 50 cm³ d'eau glacée, essoré puis séché à l'étuve sous pression réduite (10 Pa) à une température voisine de 40°C pendant 18 heures. On obtient ainsi 49,4 g de 2-(acétylamino)-2-(4-pyridinylméthyl)malonate de diéthyle, sous forme d'un solide blanc-rosé.

Spectre d'infra-rouge CH₂Cl₂ 3409; 2895; 1741; 1681; 1603; 1496; 1302; 1198; 1057; 855; 557 et 524 cm⁻¹.

### Bromhydratre de la 4-brométhyl-pyridine

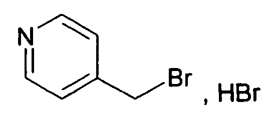

A une suspension de 140 g de bromure de triphénylphosphine dans 750 cm³ de dichlorométhane sous agitation et atmosphère inerte, on coule au goutte à goutte une solution de 30 g de 4-pyridyl-carbinol dans 250 cm³ de dichlorométhane en maintenant une température inférieure à 27°C. Le milieu réactionnel est agité à une température voisine de 20°C pendant 4 heures, puis l'insoluble obtenu est filtré, rincé par 100 cm³ de dichlorométhane et essoré. On obtient ainsi 61,5 g de bromhydratre de la 4-brométhyl-pyridine, sous forme d'un solide blanc. Spectre de masse EI m/z=171 M⁺ m/z=92 C₆H₆N pic de base m/z=65 C₅H₅⁺.

Les compositions pharmaceutiques selon l'invention sont constitués par un composé de formule (I) ou un isomère ou un tautomère ou un sel d'un tel composé, à l'état pur ou sous forme d'une composition dans laquelle il est associé à tout autre produit pharmaceutiquement compatible, pouvant être inerte ou physiologiquement actif. Les médicaments selon l'invention peuvent être employés par voie orale, parentérale, rectale ou topique.

Comme compositions solides pour administration orale, peuvent être utilisés des comprimés, des pilules, des poudres (capsules de gélatine, cachets) ou des granulés. Dans ces compositions, le principe actif selon l'invention est mélangé à un ou plusieurs diluants inertes, tels que amidon, cellulose, saccharose, lactose ou silice, sous courant d'argon. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou d'autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration rectale sont les suppositoires ou les capsules rectales qui contiennent, outre le produit actif, des excipients tels que le beurre de cacao, des glycérides semi-synthétiques ou des polyéthylèneglycols.

Les compositions pour administration topique peuvent être par exemple des crèmes, lotions, collyres, collutoires, gouttes nasales ou aérosols.

En thérapeutique humaine, les composés selon l'invention sont particulièrement utiles pour le traitement et/ou la prévention de la sclérose en plaques, l'ischémie cérébrale focale ou globale, les traumatismes cérébraux ou spinaux, la maladie de Parkinson, la maladie de Huntington, la maladie d'Alzheimer, la sclérose latérale amyotrophique, la migraine, la dépression, la schizophrénie, l'anxiété, l'épilepsie, le diabète, l'athérosclérose, la myocardite, l'arthrite, l'arthrose, l'asthme, le syndrome du colon irritable, la maladie de Crohn, la péritonite, le reflux gastro-oesophagien, l'uvéite, le syndrome de Guillain-Barré, la glomérulo-néphrite, le lupus érythémateux, le psoriasis, la croissance de certaines formes de tumeurs comme par exemple les épithéliomes, les adénocarcinomes ou les sarcomes, et les infections par des bactéries intracellulaires ou extracellulaires, Gram-plus ou Gram-moins.

Les doses dépendent de l'effet recherché, de la durée du traitement et de la voie d'administration utilisée; elles sont généralement comprises entre 1 mg et 100 mg par jour par voie orale pour un adulte avec des doses unitaires allant de 0,5 mg à 50 mg de substance active.

D'une façon générale, le médecin déterminera la posologie appropriée en fonction de l'âge, du poids et de tous les autres facteurs propres au sujet à traiter.

Les exemples suivants illustrent des compositions selon l'invention :

### EXEMPLE A

On prépare, selon la technique habituelle, des gélules dosées à 50 mg de produit actif ayant la composition suivante :
- Composé de formule (I) 50 mg
- Cellulose 18
- Lactose 55 mg
- Silice colloïdale 1 mg
- Carboxyméthylamidon sodique 10 mg
- Talc 10 mg
- Stéarate de magnésium 1 mg

### EXEMPLE B

On prépare selon la technique habituelle des comprimés dosés à 50 mg de produit actif ayant la composition suivante :
- Composé de formule (I) 50 mg
- Lactose 104 mg
- Cellulose 40 mg
- Polyvidone 10 mg
- Carboxyméthylamidon sodique 22 mg
- Talc 10 mg
- Stéarate de magnésium 2 mg
- Silice colloïdale 2 mg
- Mélange d'hydroxyméthylcellulose, glycérine, oxyde de titane (72-3,5-24,5) q.s.p. 1 comprimé pelliculé terminé à 245 mg

### EXEMPLE C

On prépare une solution injectable contenant 10 mg de produit actif ayant la composition suivante :
- Composé de formule (I) 10 mg
- Acide benzoïque 80 mg
- Alcool benzylique 0,06 ml
- Benzoate de sodium 80 mg
- Ethanol à 95 % 0,4 ml
- Hydroxyde de sodium 24 mg
- Propylène glycol 1,6 ml
- Eau q.s.p. 4 ml

La présente invention concerne également la méthode de prévention et de traitement des maladies dans lesquelles une production anormale de monoxyde d'azote (NO) par induction de NO-synthase inductible (NOS-2 ou iNOS) est impliquée par administration d'un composé de formule (I) ses racémiques, énantiomères, diastéréoisomères et leurs mélanges, son tautomère et ses sels pharmaceutiquement acceptables.

## Revendications

1. Composés de formule (I) 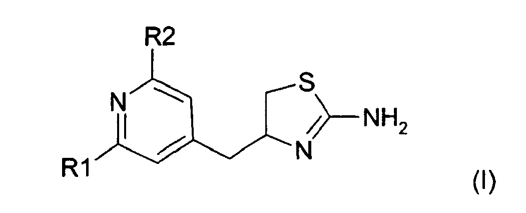 dans laquelle
soit R1, R2 sont identiques et représentent un radical hydroxy, alkyle(C1-C4) un chlore, ou un alcoxy(C1-C4);
soit au moins un des deux R1, R2 est un hydrogène et l'autre est un radical alkyle(C1-C4), alcoxy(C1-C4), un hydroxy ou un chlore
étant entendu que les radicaux alkyle(C1-C4) et alcoxy(C1-C4) renferment 1 à 4 carbones en chaîne droite ou ramifiée
leurs racémiques, énantiomères, diastéréoisomères et leurs mélanges, leurs tautomères et leurs sels pharmaceutiquement acceptables.

2. Composé selon la revendication 1 **caractérisé par le fait qu'**il est choisi parmi :
4-(2-Hydroxy-pyridin-4-ylméthyl)-4,5-dihydro-thiazol-2-ylamine
4-(2-chloro-pyridin-4-ylméthyl)-4,5-dihydro-1,3-thiazol-2-ylamine
4-(2,6-dichloro-pyridin-4-ylméthyl)-4,5-dihydro-1,3-thiazol-2-ylamine
leur racémiques, énantiomères, diastéréoisomères et leurs mélanges, leurs tautomères et leurs sels pharmaceutiquement acceptables.

3. Composé selon l'une quelconque des revendications 1 à 2 **caractérisé par le fait qu'**il est choisi parmi :
(+)-4-(2-Hydroxy-pyridin-4-ylméthyl)-4,5-dihydro-thiazol-2-ylamine
4-(2-chloro-pyridin-4-ylméthyl)-4,5-dihydro-1,3-thiazol-2-ylamine
4-(2,6-dichloro-pyridin-4-ylméthyl)-4,5-dihydro-1,3-thiazol-2-ylamine
leurs tautomères ainsi que leurs sels pharmaceutiquement acceptables.

4. Composé selon l'une quelconque des revendications 1 à 3 utilisé pour préparer un médicament.

5. Composition pharmaceutique **caractérisée par le fait qu'**elle comprend dans un milieu pharmaceutiquement acceptable, un composé défini selon l'une quelconque des revendications 1 à 3.

6. Médicaments selon la revendication 4 **caractérisés par le fait qu'**il contient au moins un composé défini selon l'une quelconque des revendications 1 à 3 pour son application thérapeutique dans le traitement des maladies dans lesquelles une production anormale de monoxyde d'azote (NO) par induction de NO-synthase inductible (NOS-2) est impliquée.

7. Médicaments selon la revendication 4 **caractérisés par le fait qu'**il contient au moins un composé défini selon l'une quelconque des revendications 1 à 3 pour son application thérapeutique dans le traitement de la maladie de Parkinson.

8. Procédé de préparation des composés de formule (I) tels que définis dans la revendication 1 **caractérisé en ce que** l'on cyclise un dérivé de formule : 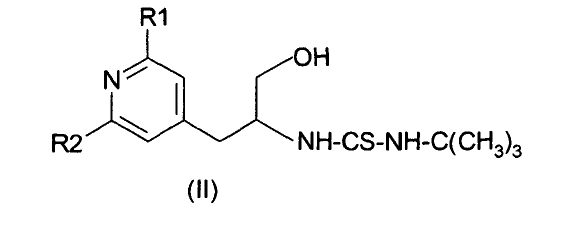 dans laquelle R1 et R2 ont les mêmes significations que dans la revendication 1, et le transforme éventuellement en sel pharmaceutiquement acceptable.

9. Procédé de préparation selon la revendication 8 **caractérisé en ce que** la cyclisation se fait en milieu acide à une température voisine de 100°C.

10. Procédé de préparation selon la revendication 9 **caractérisé en ce que** le milieu acide est préférentiellement de l'acide chlorhydrique 6N

11. Procédé de préparation des composés de formule (II) telle que définie à la revendication 8 et dans laquelle R1 et R2 ont la même signification qu'à la revendication 1 **caractérisé en ce qu'**on soumet un composé de formule (IIa) 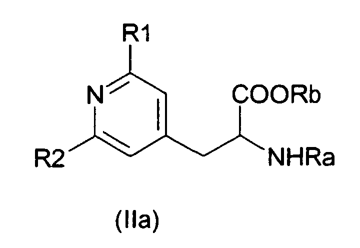 Ra est un groupe protecteur de la fonction amine tel que CO₂ tBu ou Ac et Rb étant soit un radical alkyle (1-4C), soit un radical alkoxycarbonyle à l'action d'un agent de réduction afin d'obtenir le composé de formule (IIb) 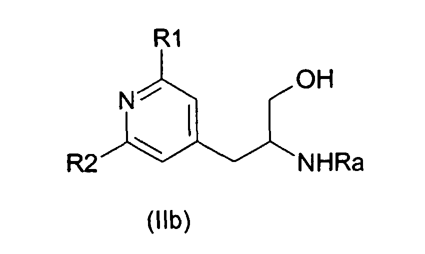 que l'on soumet à l'action d'un agent de déprotection afin d'obtenir un composé de formule (IIc) 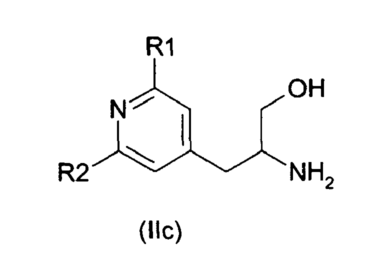 que l'on soumet à l'action du *tert*-butylisothiocyanate afin d'obtenir un composé de formule (II)

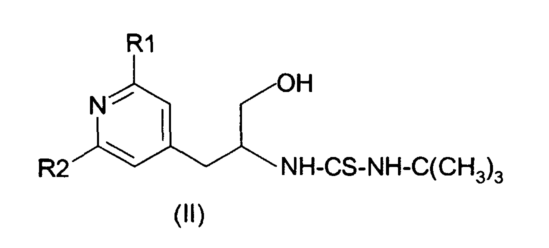

12. Procédé de préparation des composés de formule (I) tels que définis dans la revendication 1 pour lesquels R1 est OH et R2 est H **caractérisé en ce que** l'on cyclise un dérivé de formule : 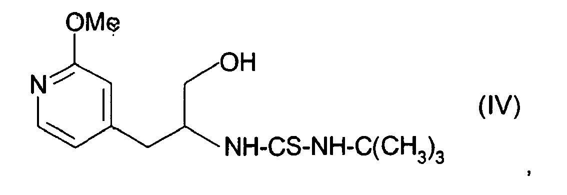 et le transforme éventuellement en sel pharmaceutiquement acceptable.

13. Procédé de préparation selon la revendication 12 **caractérisé en ce que** la cyclisation se fait en milieu acide à une température voisine de 100°C.

14. Procédé de préparation selon la revendication 13 **caractérisé en ce que** le milieu acide est préférentiellement de l'acide chlorhydrique 6N

15. Procédé de préparation des composés de formule (IV) selon la revendication 12 **caractérisé en ce que** l'on soumet un composé de formule (V) : 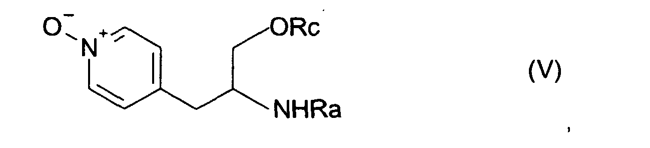 dans laquelle Ra est un groupement protecteur de la fonction amine et Rc est un groupement protecteur de la fonction alcool tel que un radical acetyl ou tert-butoxycarbonyle pour Ra et tel que *tert*-butyldiméthylsilyle pour Rc à l'action du chlorure de *para*-toluènesulfonyle en présence d'une base dans un solvant alcoolique afin d'obtenir un composé de formule (VI) 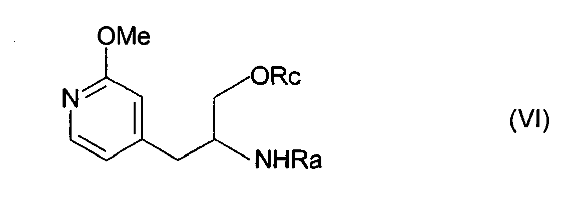 que l'on soumet à l'action d'un agent de déprotection afin d'obtenir un composé de formule (VII) 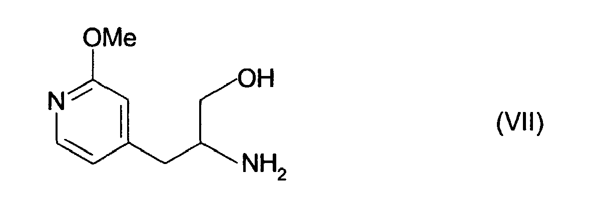 que l'on soumet à l'action du *tert*-butylisothiocyannate afin d'obtenir un composé de formule (IV) telle que défini à la revendication 12.

16. A titre de produits intermédiaires, les composés formules IV, V, VI, VII telles que définies à la revendication 15.

17. A titre de produits intermédiaires, les composée de formules II, IIa, IIb, IIc telle que définies dans la revendication 11 à l'exclusion de 2-Acétylamino-3-(2-éthoxy-pyridin-4-yl)-propionate d'éthyle.

18. A titre de produits intermédiaires, les composés *N*-(*tert*-Butyl)-N'-[2-hydroxy-1-(3-méthoxy-pyridin-4-yl-méthyl)éthyl]-thiourée ; 2-amino-3-(3-méthoxy-pyridin-4-yl)-1-propanol ; N-[1-(tert-Butyl-diméthyl-silanyloxyméthyl)-2-(3-méthoxy-pyridin-4-yl)éthyl]-acétamide ; *N*-[[1-(*tert*-Butyl-diméthyl-silanyloxyméthyl)-2-(1-oxy-pyridin-4-yl)-éthyl]-acétamide

## Claims

1. Compounds of formula (I) 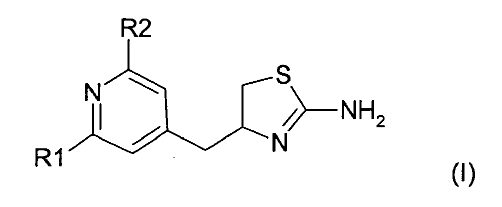 in which
either R1 and R2 are identical and represent a hydroxyl or (C1-C4)alkyl radical, a chlorine or a (C1-C4)alkoxy;
or at least one of the two, R1, R2, is a hydrogen and the other is a (C1-C4)alkyl or (C1-C4)alkoxy radical, a hydroxyl or a chlorine
with the proviso that the (C1-C4)alkyl and (C1-C4)alkoxy radicals contain 1 to 4 carbons in a straight or branched chain
their racemic mixtures, enantiomers, diastereoisomers and mixtures thereof, their tautomers and their pharmaceutically acceptable salts.

2. Compound according to Claim 1, **characterized in that** it is selected from:
4-(2-hydroxy-pyridin-4-ylmethyl)-4,5-dihydro-thiazol-2-ylamine
4-(2-chloro-pyridin-4-ylmethyl)-4,5-dihydro-1,3-thiazol-2-ylamine
4-(2,6-dichloro-pyridin-4-ylmethyl)-4,5-dihydro-1, 3-thiazol-2-ylamine
their racemic mixtures, enantiomers, diastereoisomers and mixtures thereof, their tautomers and their pharmaceutically acceptable salts.

3. Compound according to either of Claims 1 and 2, **characterized in that** it is selected from:
(+)-4-(2-hydroxy-pyridin-4-ylmethyl)-4,5-dihydro-thiazol-2-ylamine
4-(2-chloro-pyridin-4-ylmethyl)-4,5-dihydro-1,3-thiazol-2-ylamine
4-(2,6-dichloro-pyridin-4-ylmethyl)-4,5-dihydro-1,3-thiazol-2-ylamine
their tautomers and their pharmaceutically acceptable salts.

4. Compound according to any one of Claims 1 to 3, used to prepare a medicament.

5. Pharmaceutical composition, **characterized in that** it comprises in a pharmaceutically acceptable medium a compound defined according to any one of Claims 1 to 3.

6. Medicament according to Claim 4, **characterized in that** it contains at least one compound defined according to any one of Claims 1 to 3 for its therapeutic application in the treatment of diseases involving abnormal production of nitrogen monoxide (NO) by induction of inducible NO synthase (NOS-2).

7. Medicament according to Claim 4, **characterized in that** it contains at least one compound defined according to any one of Claims 1 to 3 for its therapeutic application in the treatment of Parkinson's disease.

8. Process for preparing the compounds of formula (I) as defined in Claim 1, **characterized in that** a derivative of formula: 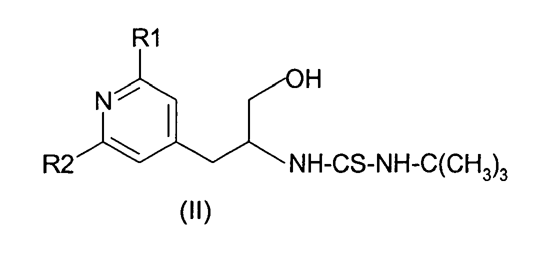 in which R1 and R2 have the same meanings as in Claim 1 is cyclized and is converted optionally into a pharmaceutically acceptable salt.

9. Preparation process according to Claim 8, **characterized in that** the cyclization takes place in an acidic medium at a temperature in the region of 100°C.

10. Preparation process according to Claim 9, **characterized in that** the acidic medium is preferably 6N hydrochloric acid

11. Process for preparing the compounds of formula (II) as defined in Claim 8 and in which R1 and R2 have the same meaning as in Claim 1, **characterized in that** a compound of formula (IIa) 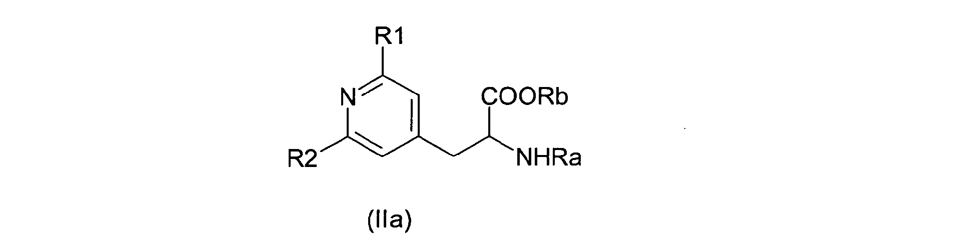 Ra is a protective group for the amine function such as CO₂ tBu or Ac and Rb being either a (1-4C) alkyl radical or an alkoxycarbonyl radical is subjected to the action of a reducing agent so as to give the compound of formula (IIb) 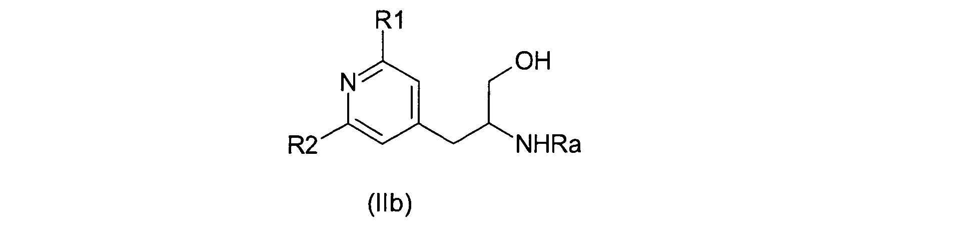 which is subjected to the action of a deprotection agent so as to give a compound of formula (IIc) 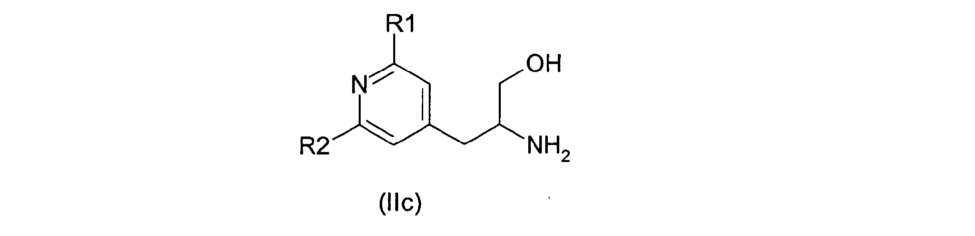 which is subjected to the action of *tert*-butyl isothiocyanate so as to give a compound of formula (II)

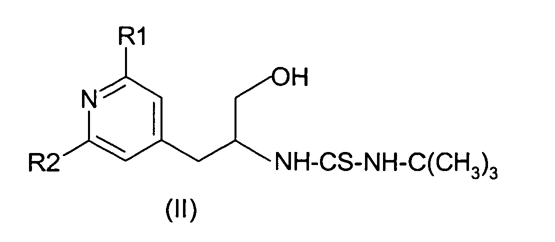

12. Process for preparing the compounds of formula (I) as defined in Claim 1 for which R1 is OH and R2 is H, **characterized in that** a derivative of formula: 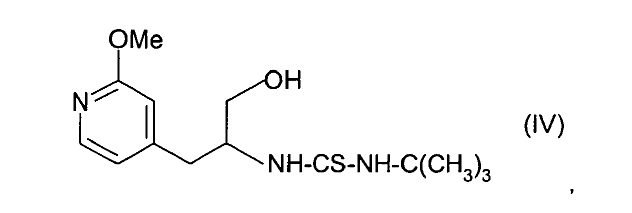 is cyclized and is converted optionally into a pharmaceutically acceptable salt.

13. Preparation process according to Claim 12, **characterized in that** the cyclization takes place in an acidic medium at a temperature in the region of 100°C.

14. Preparation process according to Claim 13, **characterized in that** the acidic medium is preferably 6N hydrochloric acid.

15. Process for preparing the compounds of formula (IV) according to Claim 12, **characterized in that** a compound of formula (V): 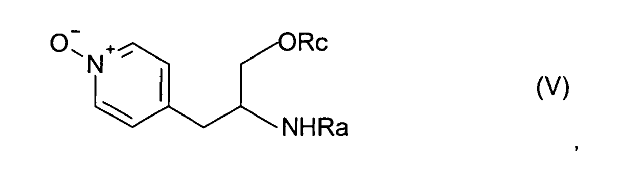 in which Ra is a protective group for the amine function and Rc is a protective group for the alcohol function such as an acetyl or tert-butoxycarbonyl radical for Ra and such as *tert*-butyldimethylsilyl for Rc, is subjected to the action of *para-*toluenesulphonyl chloride in the presence of a base in an alcoholic solvent so as to give a compound of formula (VI) 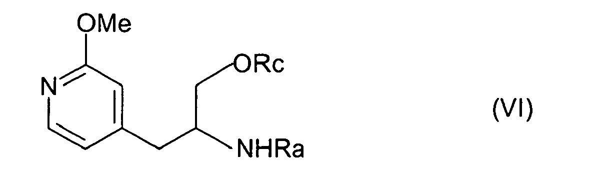 which is subjected to the action of a deprotection agent so as to give a compound of formula (VII) 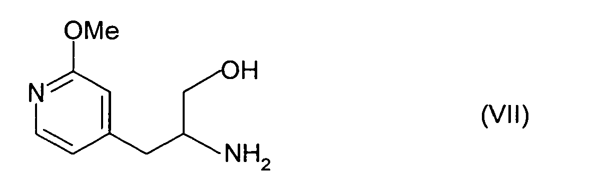 which is subjected to the action of *tert*-butyl isothiocyanate so as to give a compound of formula (IV) as defined in Claim 12.

16. As intermediates, the compounds of formulae IV, V, VI and VII as defined in Claim 15.

17. As intermediates, the compounds of formulae II, IIa, IIb and IIc as defined in Claim 11 with the exception of ethyl 2-acetylamino-3-(2-ethoxypyridin-4-yl)propionate.

18. As intermediates, the compounds *N*-(*tert*-butyl)-N'-[2-hydroxy-1-(3-methoxypyridin-4-yl-methyl)ethyl]thiourea; 2-amino-3-(3-methoxypyridin-4-yl)-1-propanol; N-[1-(tert-butyldimethyl-silanyloxymethyl)-2-(3-methoxypyridin-4-yl)ethyl]-acetamide; *N*-[1-(*tert*-butyldimethyl-silanyloxymethyl)-2-(1-oxypyridin-4-yl)ethyl]-acetamide.

## Patentansprüche

1. Verbindungen der Formel (I) 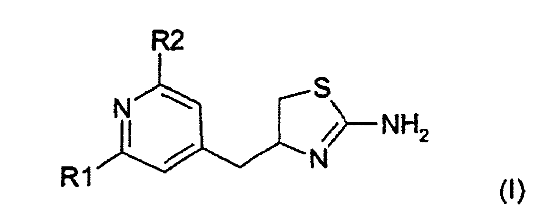 worin
entweder R1 und R2 gleich sind und für einen Hydroxy- oder (C1-C4)-Alkylrest, Chlor oder (C1-C4)-Alkoxy stehen
oder mindestens eine der beiden Gruppen R1 und R2 für Wasserstoff steht und die andere für einen (C1-C4)-Alkyl- oder (C1-C4)-Alkoxyrest, Chlor oder Hydroxy steht,
mit der Maßgabe, daß die (C1-C4)-Alkyl- und (C1-C4)-Alkoxyreste 1 bis 4 Kohlenstoffe in gerader oder verzweigter Kette enthalten,
Racemate, Enantiomere, Diastereomere und Mischungen davon, Tautomere davon und pharmazeutisch unbedenkliche Salze davon.

2. Verbindung nach Anspruch 1, **dadurch gekennzeichnet, daß** sie unter:
4-(2-Hydroxypyridin-4-ylmethyl)-4,5-dihydrothiazol-2-ylamin
4-(2-Chlorpyridin-4-ylmethyl)-4,5-dihydro-1,3-thiazol-2-ylamin
4-(2,6-Dichlorpyridin-4-ylmethyl)-4,5-dihydro-1,3-thiazol-2-ylamin
ausgewählt ist,
Racemate, Enantiomere, Diastereomere und Mischungen davon, Tautomere davon und pharmazeutisch unbedenkliche Salze davon.

3. Verbindung nach einem der Ansprüche 1 bis 2, **dadurch gekennzeichnet, daß** sie unter:
(+)-4-(2-Hydroxypyridin-4-ylmethyl)-4,5-dihydrothiazol-2-ylamin
4-(2-Chlorpyridin-4-ylmethyl)-4,5-dihydro-1,3-thiazol-2-ylamin
4-(2,6-Dichlorpyridin-4-ylmethyl)-4,5-dihydro-1,3-thiazol-2-ylamin
ausgewählt ist,
Tautomere davon und pharmazeutisch unbedenkliche Salze davon.

4. Verbindung nach einem der Ansprüche 1 bis 3, die zur Herstellung eines Arzneimittels verwendet wird.

5. Pharmazeutische Zusammensetzung, **dadurch gekennzeichnet, daß** sie eine Verbindung gemäß einem der Ansprüche 1 bis 3 in einem pharmazeutisch unbedenklichen Medium umfaßt.

6. Arzneimittel nach Anspruch 4, **dadurch gekennzeichnet, daß** sie mindestens eine Verbindung gemäß einem der Ansprüche 1 bis 3 für deren therapeutische Anwendung bei der Behandlung von Erkrankungen, an denen eine anormale Produktion von Stickstoffmonoxid (NO) durch Induktion von induzierbarer NO-Synthase (NOS-2) beteiligt ist, enthalten.

7. Arzneimittel nach Anspruch 4, **dadurch gekennzeichnet, daß** sie mindestens eine Verbindung gemäß einem der Ansprüche 1 bis 3 für deren therapeutische Anwendung bei der Behandlung von Parkinsonscher Krankheit enthalten.

8. Verfahren zur Herstellung von Verbindungen der Formel (I) gemäß Anspruch 1, **dadurch gekennzeichnet, daß** man ein Derivat der Formel: 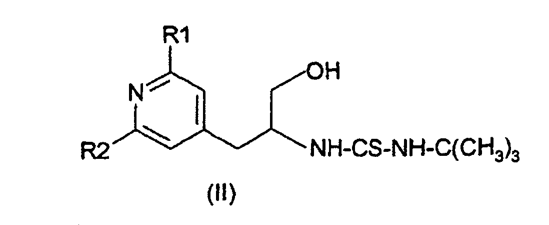 worin R1 und R2 die gleichen Bedeutungen wie in Anspruch 1 besitzen, cyclisiert und gegebenenfalls in ein pharmazeutisch unbedenkliches Salz umwandelt.

9. Herstellungsverfahren nach Anspruch 8, **dadurch gekennzeichnet, daß** die Cyclisierung in saurem Medium bei einer Temperatur in der Nähe von 100°C stattfindet.

10. Herstellungsverfahren nach Anspruch 9, **dadurch gekennzeichnet, daß** es sich bei dem sauren Medium vorzugsweise um 6 N Salzsäure handelt.

11. Verfahren zur Herstellung von Verbindungen der Formel (II) gemäß Anspruch 8, worin R1 und R2 die gleiche Bedeutung wie in Anspruch 1 besitzen, **dadurch gekennzeichnet, daß** man eine Verbindung der Formel (IIa) 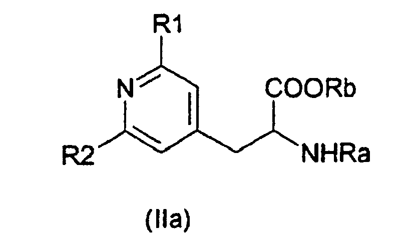 worin Ra für eine Schutzgruppe der Aminfunktion wie CO₂tBu oder Ac steht und Rb entweder für einen (C1-C4)-Alkylrest oder einen Alkoxycarbonylrest steht, der Einwirkung eines Reduktionsmittels unterwirft, wodurch man die Verbindung der Formel (IIb) 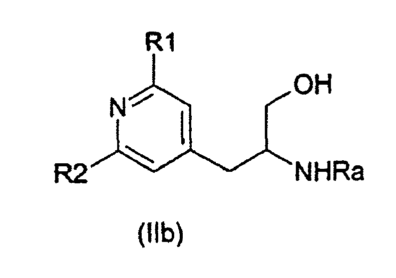 erhält, welche man der Einwirkung eines Entschützungsmittels unterwirft, wodurch man eine Verbindung der Formel (IIc) 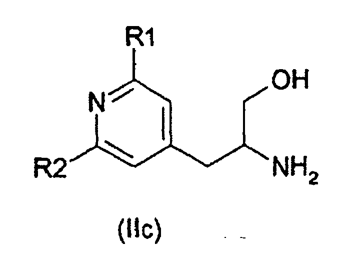 erhält, welche man der Einwirkung von tert.-Butyl-isothiocyanat unterwirft, wodurch man eine Verbindung der Formel (II) 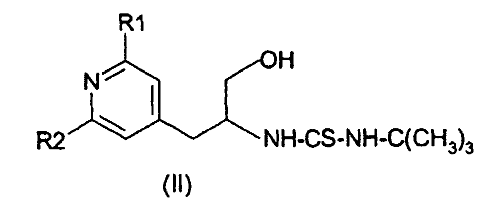 erhält.

12. Verfahren zur Herstellung von Verbindungen der Formel (I) gemäß Anspruch 1, worin R1 für OH steht und R2 für H steht, **dadurch gekennzeichnet, daß** man ein Derivat der Formel: 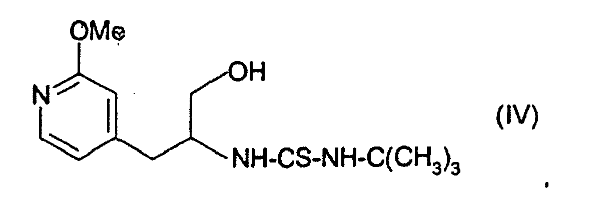 cyclisiert und gegebenenfalls in ein pharmazeutisch unbedenkliches Salz umwandelt.

13. Herstellungsverfahren nach Anspruch 12, **dadurch gekennzeichnet, daß** die Cyclisierung in saurem Medium bei einer Temperatur in der Nähe von 100°C stattfindet.

14. Herstellungsverfahren nach Anspruch 13, **dadurch gekennzeichnet, daß** es sich bei dem sauren Medium vorzugsweise um 6 N Salzsäure handelt.

15. Verfahren zur Herstellung von Verbindungen der Formel (IV) nach Anspruch 12, **dadurch gekennzeichnet, daß** man eine Verbindung der Formel (V): 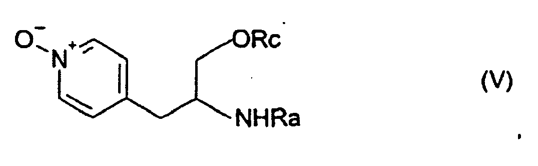 worin Ra für eine Schutzgruppe der Aminfunktion steht und Rc für eine Schutzgruppe der Alkoholfunktion steht, wie ein Acetyl- oder tert.-Butoxycarbonylrest für Ra und wie *tert*.-Butyldimethylsilyl für Rc, der Einwirkung von *para*-Toluolsulfonylchlorid in Gegenwart einer Base in einem alkoholischen Lösungsmittel unterwirft, wodurch man eine Verbindung der Formel (VI) 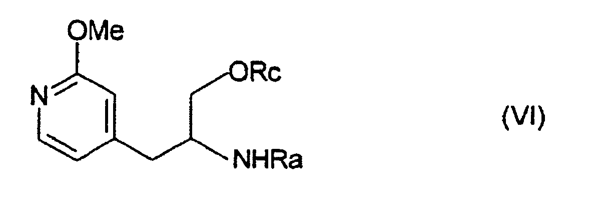 erhält, welche man der Einwirkung eines Entschützungsmittels unterwirft, wodurch man eine Verbindung der Formel (VII) 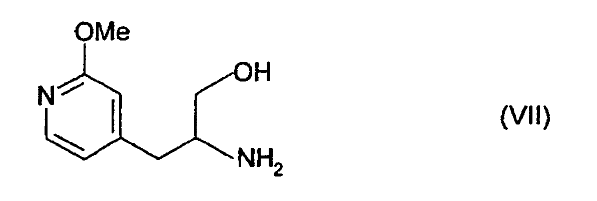 erhält, welche man der Einwirkung von *tert*.-Butyl-isothiocyanat unterwirft, wodurch man eine Verbindung der Formel (IV) gemäß Anspruch 12 erhält.

16. Als Zwischenprodukte die Verbindungen der Formeln IV, V, VI und VII gemäß Anspruch 15.

17. Als Zwischenprodukte die Verbindungen der Formeln II, IIa, IIb und IIc gemäß Anspruch 11 mit Ausnahme von 2-Acetylamino-3-(2-ethoxypyridin-4-yl)propionsäureethylester.

18. Als Zwischenprodukte die Verbindungen *N*-(*tert*.-Butyl)-N'-[2-hydroxy-1-(3-methoxypyridin-4-yl-methyl)ethyl]thioharnstoff; 2-Amino-3-(3-methoxypyridin-4-yl)-1-propanol; N-[1-(tert.-Butyl-dimethylsilanyloxymethyl)-2-(3-methoxypyridin-4-yl)ethyl]acetamid und *N*-[1-(*tert*.-Butyldimethylsilanyloxymethyl)-2-(1-oxypyridin-4-yl)ethyl]acetamid.
